(19) Europäisches Patentamt
European Patent Office
Office européen des brevets

(11) Publication number: 0 094 161 A1

# (12) EUROPEAN PATENT APPLICATION

(21) Application number: 83302145.4

(22) Date of filing: 15.04.83

(51) Int. Cl.³: C 12 Q 1/54, C 12 N 9/04

(30) Priority: 07.05.82 GB 8213207

(43) Date of publication of application: 16.11.83 Bulletin 83/46

(84) Designated Contracting States: BE CH DE FR GB IT LI NL SE

(71) Applicant: IMPERIAL CHEMICAL INDUSTRIES PLC, Imperial Chemical House Millbank, London SW1P 3JF (GB)

(72) Inventor: Senior, Peter James, Foulis Cottage Ingleby Greenhow, Middlesbrough Cleveland (GB)
Inventor: Collins, Stephen Hugh, "Ravensdown" Clack Bank Osmotherley, Northallerton North Yorkshire (GB)

(74) Representative: Aufflick, James Neil et al, Imperial Chemical Industries PLC Legal Department: Patents Thames House North Millbank, London SW1P 4QG (GB)

## (54) Method for determining glucose content of fluid.

(57) A method for determining glucose present in a fluid which uses an assay mixture comprising a pyrroloquinone-dependent glucose dehydrogenase enzyme E.C. No. 1.1.99.17 or a flavin-dependent glucose dehydrogenase enzyme E.C. No. 1.1.99.10 and a reducible compound, reduction of which can produce changes in electro-magnetic radiation absorbance characteristics and/or electrical changes. The method is particularly applicable to the determination of glucose in physiological fluids such as blood. A glucose assay mixture is also claimed.

EP 0 094 161 A1

Method for determining glucose content of fluid

This invention relates to a method for determining the glucose content of a fluid and to a glucose assay mixture which can be used in the method. In particular the invention relates to a method and a glucose assay mixture for determining the glucose content of physiological fluids such as blood.

The measurement of glucose in physiological fluids such as blood is carried out extensively in hospitals. Several assay methods are available for this including enzymatic colorimetric and chemical methods. Enzymatic assay methods are preferred. The enzymatic method most favoured at present uses the enzymes glucose oxidase and peroxidase and is dependent upon measuring liberated hydrogen peroxide. This method has a number of disadvantages including (a) it is complicated, requiring two enzymes, and (b) it is subject to possible interference arising either from decomposition of hydrogen peroxide by catalase from samples or non-specific reactions of the peroxidase such that the hydrogen peroxide reacts with serum components rather than in the required manner.

According to the present invention we provide a method for determining glucose present in a fluid wherein a sample of a glucose-containing fluid is contacted with an assay mixture comprising a pyrroloquinone-dependent glucose dehydrogenase enzyme E.C. (Enzyme Commission) No. 1.1.99.17 or a flavin-dependent glucose dehydrogenase enzyme E.C. (Enzyme Commission) No. 1.1.99.10 and a reducible compound, reduction of which can produce changes

in electro-magnetic radiation absorbance characteristics and/or electrical changes, in an appropriate buffered medium.

Further according to the invention we provide a glucose assay mixture which comprises a pyrroloquinone-dependent glucose dehydrogenase enzyme E.C. (Enzyme Commission) No. 1.1.99.17 or a flavin-dependent glucose dehydrogenase enzyme E.C. (Enzyme Commission) No. 1.1.99.10 and a reducible compound, reduction of which can produce changes in electro-magnetic radiation absorbance characteristics and/or electrical changes.

The E.C. (Enzyme Commission) Nos. specified above are enzyme numbers assigned to enzymes in the Enzyme Nomenclature of the Nomenclature Committee of the International Union of Biochemistry (NC-IUB).

Suitably the reducible compound is one whose reduction may be monitored either by measuring a change in the electro-magnetic radiation absorbance characteristics and/or by electronic means whereby a flow of electrons may be transferred from the compound to an electrode system thus producing an electric current. When the reducible compound is one whose reduction is monitored by measuring a change in electro-magnetic radiation obsorbance characteristics (e.g. spectrophotometrically) that compound will be a dye. Certain compounds may be usable as reducible compounds in either respect, i.e. may be dyes and also compounds whose reduction can produce electrical changes to be monitored by electronic means.

The method of the invention can be used to determine the concentration of glucose in any glucose-containing fluid. In particular the method is useful for determing the glucose concentration in blood and in other physiological fluids. It is however useful for determining the glucose concentration in a wide range of other glucose-containing fluids including industrial process fluids, for example in processes for the production of glucose isomerase, and waste water.

The method of the invention employs a reaction mechanism in which the enzyme glucose dehydrogenase effects oxidation of

glucose to gluconolactone which is subsequently hydrolysed to gluconic acid. This reaction mechanism releases two electrons from each molecule of glucose. The electrons released from glucose molecules pass to molecules of the reducible compound which is thus reduced and provide the basis for the absorbance and/or electrical changes which enable the reaction to be monitored. For instance in the presence of a suitable dye compound the released electrons from glucose molecules reduce molecules of the dye compound thereby producing a change in the electro-magnetic radiation absorbance characteristics of the medium which can be measured using e.g. a spectrophotometer. Preferably the change in absorbance characteristics is a colour change measureable in the visible region of the spectrum. The extent of the change in absorbance characteristics can be determined using a spectrophotometer to measure the change in optical density at a suitable wavelength. For direct electronic detection, the reaction mixture contains an inert solid electrode (e.g. platinum or suitably treated carbon) which is capable of accepting electrons from the reducible compound and which forms one electrode of a cell. The cell is connected by a second electrode at which a reduction reaction can occur (e.g. a silver/silver chloride, standard calomel electrode or platinum in ferricyanide). A salt bridge makes a liquid connection between the two electrode systems. The rate of the glucose oxidation reaction may be monitored directly amperometrically. Alternatively the change in concentrations of the reduced and oxidised form of the reducible compound may be followed potentiometrically.

Preferably the method of the invention is carried out using the assay mixture of the invention. This can be done by taking a prepared assay mixture and adding it to the glucose-containing fluid under examination, buffering the medium in a suitable manner. Alternatively the assay mixture can be made up *in situ* by adding the components to the glucose-containing fluid and buffering.

The glucose dehydrogenase enzymes may be obtained from

any microorganism containing these enzymes. Microorganisms containing these enzymes and which can usefully be used as sources for them include strains of Acetobacter, Acinetobacter, Gluconobacter and Pseudomonas as sources of E.C. 1.1.99.17 and Aspergillus as sources of E.C. 1.1.99.10. A preferred source of E.C. 1.1.99.17 is a strain of the species Acinetobacter calcoaceticus particularly strain NCTC 7844 (this strain is deposited at and is freely available from the National Collection of Type Cultures NCTC, Central Public Health Laboratory, Colindale Avenue, London). Preferred sources of E.C. 1.1.99.10 include Aspergillus oryzae strain ATCC 9102 which is deposited at and freely available from the American Type Culture Collection ATCC, 12301 Parklawn Drive, Rockville, Maryland 20852, USA.

When used in the method of the invention the enzyme may be in any form, e.g. purified enzyme, in a crude cellular extract or contained in whole microorganism cells. It may be immobilised in whole cells, immobilised in columns, used as a soluble enzyme or in any other suitable manner.

The glucose dehydrogenase enzyme E.C. 1.1.99.17 is a pyrroloquinone-dependent enzyme and contains a pyrroloquinone as a prosthetic group within it. The pyrroloquinone is a compound containing the structure:

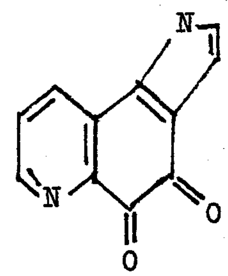

Usually the pyrroloquinone has a structure similar to that of methoxatin i.e.

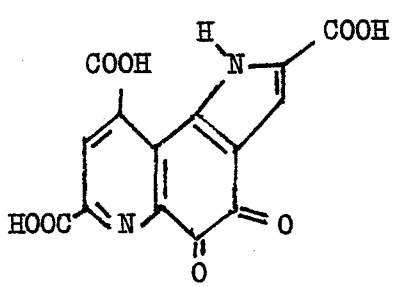

The glucose dehydrogenase enzyme E.C. 1.1.99.10 is a flavin-dependent enzyme and contains the prosthetic group flavin adenine dinucleotide (FAD).

The reducible compound can be any suitable compound which can be reduced by the electrons liberated by the glucose-dehydrogenase catalysed oxidation of glucose and which either produces a change in electromagnetic radiation absorbance characteristics or enables electrons to be donated to a solid electrode sensor. Preferably, when a dye compound is used, the absorbance characteristics change produced is in the visible region of the electro-magnetic spectrum, i.e. between 450 nm and 700 nm. A preferred dye compound is the oxidised form of 2,6-dichlorophenol (DCPIP). Reduction of these dye compounds produces colour changes in the visible region of the spectrum which can conveniently be measured at 600 nm using a spectrophotometer. In addition to the preferred dye compounds other suitable compounds include compounds having redox potentials of less than + 100 mV.

The glucose assay mixture of the invention preferably comprises a freeze-dried mixture of glucose dehydrogenase enzyme, with a suitable dye compound and buffer. The buffer may be any suitable physiologically compatible buffer. The amounts of the various components in typical assay mixtures are in the following ranges:-

| | | |
|---|---|---|
| glucose dehydrogenase | : | 10 to 200 units/ml |
| 2,6-dichlorophenol indophenol | : | 10 - 100 µM |

buffered to pH 5.5 - 9.0

The freeze dried mixture is suitably provided in a vial which can be opened and water added to the mixture to give the concentrations indicated.

In performing the method for determining glucose of the invention the assay mixture in a suitable buffered medium is prepared and a fluid sample, e.g. a sample of blood serum, is added to it in a conventional manner. Preferably 0.5 to 50 µl of the sample (or equivalent larger volume of diluted

material is added to 0.1 to 5 ml of buffered medium. Blood serum which will be a common material to be tested is formed by removing cells from blood. After addition of the sample, e.g. of serum, to the buffered medium spectrophotometric measurements are made at a suitable wavelength (e.g. 600 nm) and the glucose content of the test sample is determined.

It is also possible to use the method of the invention with paper test strips for blood, urine or serum assays. In this instance a dye compound is used. Preferably it is a mixture of nitro-blue tetrazolium and phenazine methosulphate or phenazine ethosulphate. In the presence of glucose the nitro-blue tetrazolium changes from colourless to dark purple. Phenazine ethosulphate is a compound which can be used either as a dye compound or as a reducible compound, reduction of which can produce electrical changes.

The method of the invention has advantages in that it uses only one enzyme which need not be purified to an excessively high standard and it avoids the use of highly carcinogenic and toxic chemicals. The glucose dehydrogenase enzyme E.C. 1.1.99.17 which can be used in the method of the invention does have some lesser activity on other sugars e.g. galactose and mannose but this is not significant under the assay conditions.

The Michaelis Constant Km is that concentration of the substrate for an enzyme at which the enzyme-catalysed reaction proceeds at half its theoretical maximum rate at saturating (infinite) substrate concentration. The constant for glucose dehydrogenase E.C. 1.1.99.17 from Acinetobacter is approximately 2 mM. The constant for E.C. 1.1.99.10 from Aspergillus oryzae is approximately 20 mM. The consequence of this is that the useful range in which the reaction rate is linearly proportional to substrate concentration differs by a factor of 10 for these two enzymes. Thus when the concentrations to be determined are small it is preferred to use the enzyme having the lower Km, i.e. in this instance E.C. 1.1.99.17. Conversely when, as is often the case with serum samples, the concentrations to be determined

may be relatively high it is preferred to use the enzyme having the higher Km, i.e. in this instance E.C. 1.1.99.10.

The invention is illustrated by the following Examples:-

EXAMPLE 1

Production and use of assay mixture containing glucose dehydrogenase E.C. 1.1.99.17 derived from Acinetobacter calcoaceticus strain NCTC 7844

(a) Enzyme purification

Frozen cell pellet of *Acinetobacter calcoaceticus* strain NCTC 7844 was re-suspended in phosphate buffer (50 mM, pH 7.0) and the cells were broken by sonication (3 x two minute bursts separated by two minute intervals). The cell debris was then removed by centrifugation at 50,000 rpm for 60 minutes and the resulting liquid was dialyzed overnight against 50 mM phosphate buffer at pH 7.0 before being applied to a DEAE "Sepharose" column pre-equilibrated with the same buffer. Under these conditions glucose dehydrogenase enzyme is not bound to the column whereas most of the impurities associated with the enzyme are bound to the column. The enzyme therefore passes through the column and fractions containing the enzyme were recovered. The purified enzyme thus produced was concentrated and stored.

The crude extract before purification using the DEAE sepharose column had a specific activity of 0.68 μ mol per minute per mg protein. The purified material after the treatment described above had a specific activity of 3.2 μ mol per minute per mg protein (i.e. there was an approximately five-fold increase in specific activity as a result of purification) with a recovery of 89% of the enzyme applied to the column. The purified enzyme material was used in the production of a glucose assay mixture.

(b) glucose assay

Using purified enzyme prepared as described above, the following glucose assay mixture was prepared:-

| | | |
|---|---|---|
| glucose dehydrogenase | : | 30 m units/ml assay mixture |
| 2,6-dichlorophenol indophenol | : | 50 μM |

phosphate buffer added to pH 6: 50 mM

Water being added to give the concentrations indicated.

NB - The standard international unit of enzyme activity is 1 μ mol per minute per mg protein.

The reaction was commenced by addition of a glucose containing sample (0-0.15 mM in the assay mixture) and the rate of the reaction was determined by monitoring the absorbance at 600 nm for approximately 1 minute. The initial rate was determined by extrapolation.

The results obtained are shown graphically in Figure 1 and were obtained using a series of solutions of pure glucose in water or standardised serum samples containing known concentrations of glucose. In each case 10 μl of sample were added to a reaction volume of 1 ml. The reaction rate was directly proportional to the glucose concentration over the range studied and the results obtained with the serum samples were in good agreement with those obtained using pure glucose solutions.

In the graph shown in Figure 1 initial absorbance decrease per minute at 600 nm is plotted as ordinate against glucose concentration in mM in the assay mixture as abscissa. The points on this graph are shown as follows:-

● points obtained using glucose standards in distilled water,

■ points obtained using standard sera obtained from Burroughs Wellcome.

EXAMPLE 2

Production and use of assay mixture containing glucose dehydrogenase derived from *Aspergillus oryzae* ATCC 9102

(a) Enzyme purification

Frozen cell pellet of *Aspergillus oryzae* ATCC 9102 was re-suspended in phosphate buffer (50 nm pH 7.0) and the cells were broken by sonication with glass beads (0.5 mm diameter) in 4 two minute bursts separated by two minute intervals. Cell debris was removed by centrifugation at 50,000 rpm for 60 minutes and the resulting liquid was dialysed overnight against 50 nm phosphate

buffer pH 7.0 before application to a DEAE Sepharose column pre-equilibrated with the same buffer. The glucose dehydrogenase was not bound to the column whereas the bulk of impurities were found under these conditions. The enzyme therefore passed through the column and fractions containing the enzyme were recovered. The purified enzyme so obtained was concentrated and stored.

The crude extract before purification using the DEAE sephrose had a specific activity of 0.02 µ mol per minute per mg protein which was increased by the treatment described to 0.16 µ mol per minute per mg protein (an approximately 8 fold purification) with recovery of 92% of the enzyme applied to the column. The purified material was used in production of the glucose assay mixture.

(b) Glucose assay

Using the enzyme prepared as above the following glucose assay mixture was prepared

| | | |
|---|---|---|
| glucose dehydrogenase | : | 25 m units/ml assay mixture |
| 2,6-dichlorophenol indophenol | : | 100 µm |
| phosphate buffer (pH 6.5) | : | 50 mM |

dissolved in water to give the concentrations indicated

The reaction was initiated by addition of a glucose containing sample giving a concentration of 0 - 2 mM in the assay mixture and the reaction rate was determined by following the decrease in absorbance at 600 nm. The initial reaction rate was determined.

The results are shown graphically in Figure 2 and were obtained using a series of solutions of pure glucose in water. The reaction rate was linearly proportional to glucose concentration over the range studied and results obtained with serum samples (not shown) were in good agreement with those obtained using pure glucose.

In the graph shown in Figure 2 initial rate of decrease in absorbance per minute at 600 nm is plotted as ordinate against concentration of glucose (mM in the assay mixture) as abscissa.

PA/JNA/MP
11 April 1983

1. A method for determining glucose present in a fluid wherein a sample of a glucose-containing fluid is contacted with an assay mixture comprising a pyrroloquinone-dependent glucose dehydrogenase enzyme E.C. (Enzyme Commission) No. 1.1.99.17 or a flavin-dependent glucose dehydrogenase enzyme E.C. (Enzyme Commission) No. 1.1.99.10 and a reducible compound, reduction of which can produce changes in electro-magnetic radiation absorbance characteristics and/or electrical changes, in an appropriate buffered medium.

2. A method according to claim 1 wherein the glucose-containing fluid is a physiological fluid.

3. A method according to claim 1 or claim 2 wherein a prepared assay mixture is added to a glucose-containing fluid and the medium is buffered in a suitable manner.

4. A method according to claim 1 or claim 2 wherein the assay mixture is made up *in situ* by adding its components to the glucose-containing fluid and the medium is buffered in a suitable manner.

5. A method according to any one of claims 1 to 4 wherein the glucose dehydrogenase enzyme is E.C. No. 1.1.99.17 derived from a strain of *Acetobacter*, *Acinetobacter*, *Gluconobacter* or *Pseudomonas*.

6. A method according to claim 5 wherein the glucose dehydrogenase enzyme is derived from *Acinetobacter calcoaceticus* strain NCTC 7844.

7. A method according to any one of claims 1 to 4 wherein the glucose dehydrogenase enzyme is E.C. No. 1.1.99.10 derived from a strain of *Aspergillus*.

8. A method according to claim 7 wherein the glucose dehydrogenase enzyme is derived from *Aspergillus oryzae* strain ATCC 9102.

9. A method according to any one of the preceding claims wherein the reducible compound is a dye compound reducible to produce changes in electro-magnetic radiation absorbance characteristics.

10. A method according to claim 9 wherein the dye compound is one which is reducible to produce a change in electro-magnetic radiation absorbance characteristics in the region of the spectrum between 450 nm and 700 nm.

11. A method according to claim 10 wherein the dye compound is the oxidised form of 2,6-dichlorophenol.

12. A method according to any one of claims 9 to 11 wherein paper test strips are used for blood, urine or serum assays.

13. A method according to claim 12 wherein the dye compound is a mixture of nitro-blue tetrazolium and phenazine methosulphate or phenazine ethosulphate.

14. A method according to any one of claims 1 to 8 wherein the reducible compound is one whose reduction can produce electrical changes.

15. A method according to claim 14 wherein the electrical changes are monitored amperometrically.

16. A method according to claim 14 wherein the electrical changes are monitored potentiometrically.

17. A method according to any one of the preceding claims wherein 0.5 to 50 µl of a sample of a glucose-containing fluid or an equivalent volume of diluted material is added to 0.1 to 5 ml of buffered medium containing the assay mixture.

18. A glucose assay mixture which comprises a pyrroloquinone-dependent glucose dehydrogenase enzyme E.C. (Enzyme Commission) No. 1.1.99.17 or a flavin-dependent glucose dehydrogenase enzyme E.C. (Enzyme Commission) No. 1.1.99.10 and a reducible compound, reduction of which can prpduce changes in electro-magnetic radiation absorbance characteristics and/or electrical changes.

19. A glucose assay mixture according to claim 18 wherein the glucose dehydrogenase enzyme is E.C. No. 1.1.99.17 derived from a strain of Acetobacter, Acinetobacter, Gluconobacter or Pseudomonas.

20. A glucose assay mixture according to claim 19 wherein the glucose dehydrogenase enzyme is derived from Acinetobacter calcoaceticus NCTC 7844.

21. A glucose assay mixture according to claim 18 wherein the glucose dehydrogenase enzyme is E.C. No. 1.1.99.10 derived from a strain of Aspergillus.

22. A glucose assay mixture according to claim 21 wherein the glucose dehydrogenase enzyme is derived from Aspergillus oryzae strain ATCC 9102.

23. A glucose assay mixture according to any one of claims 18 to 22 wherein the reducible compound is a dye compound which is reducible to produce a change in electro-magnetic radiation absorbance characteristics.

24. A glucose assay mixture according to claim 23 wherein the dye compound is reducible to produce a change in electro-magnetic radiation absorbance characteristics in the region of the spectrum between 450 nm and 700 nm.

25. A glucose assay mixture according to claim 24 wherein the dye compound is the oxidised form of 2,6-dichlorophenol.

26. A glucose assay mixture according to any one of claims 18 to 22 wherein the reducible compound is one whose reduction can produce electrical changes.

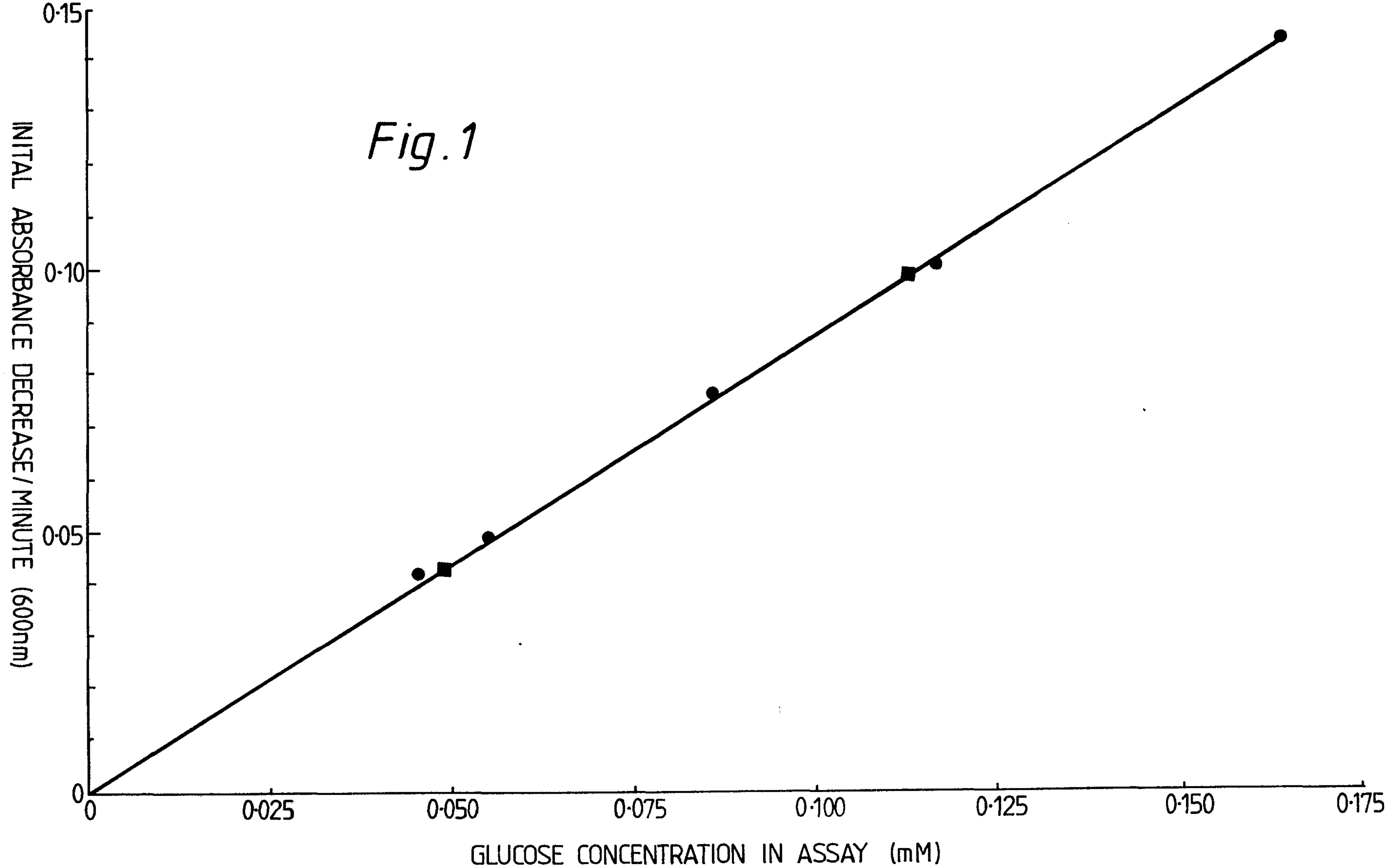
0·15
0·10
0·05
0
INITAL ABSORBANCE DECREASE/MINUTE (600nm)
0
0·025
0·050
0·075
0·100
0·125
0·150
0·175
GLUCOSE CONCENTRATION IN ASSAY (mM)

Fig. 1

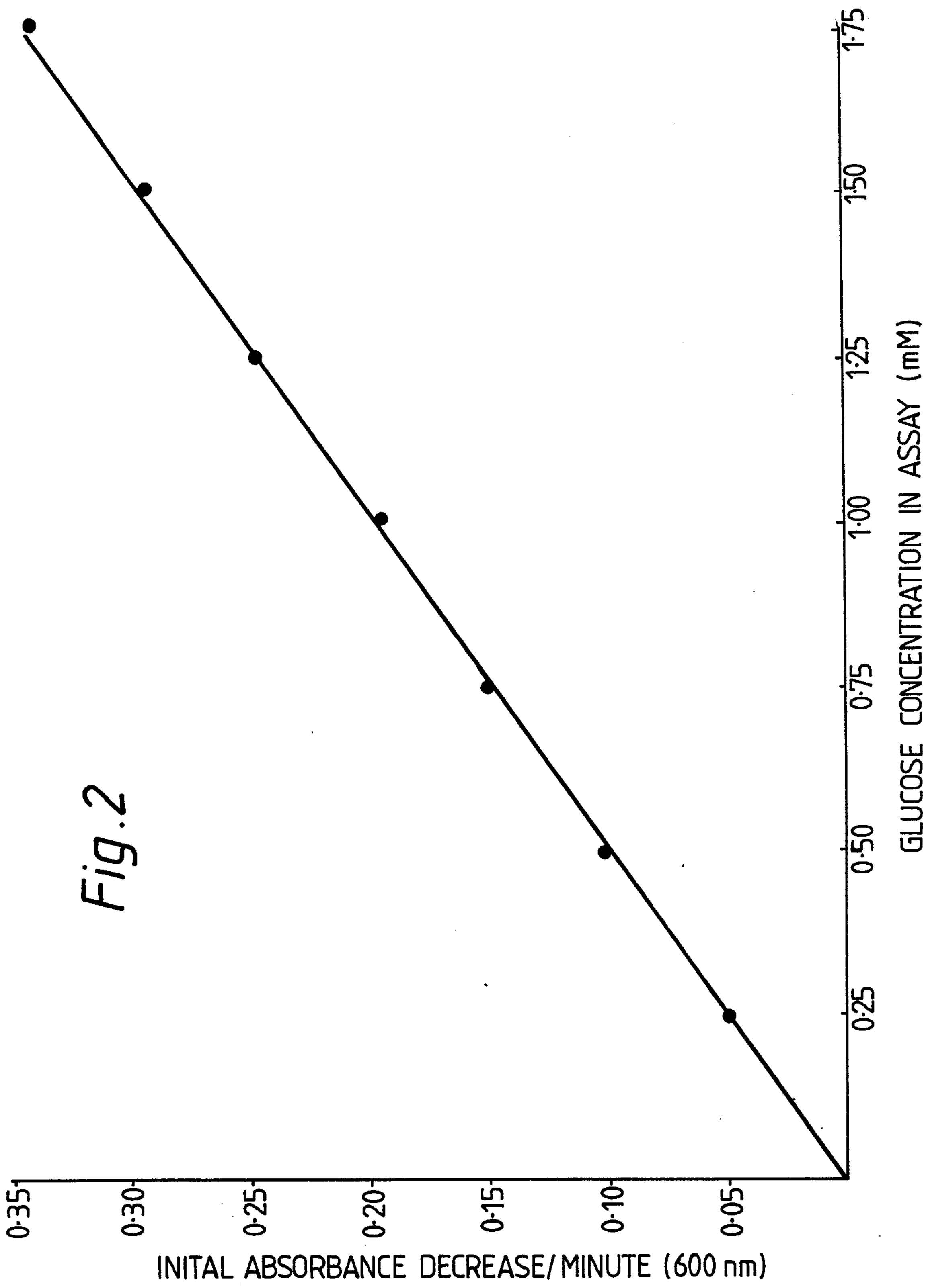
INITAL ABSORBANCE DECREASE/MINUTE (600 nm)
0·05
0·10
0·15
0·20
0·25
0·30
0·35
GLUCOSE CONCENTRATION IN ASSAY (mM)
0·25
0·50
0·75
1·00
1·25
1·50
1·75

Fig. 2

0094161

European Patent Office

# EUROPEAN SEARCH REPORT

Application number
EP 83 30 2145

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| X | AGRICULTURAL & BIOLOGICAL CHEMISTRY, vol. 44, no. 7, July 1980, pages 1505-1512, Tokyo, JP.<br>K. MATSUSHITA et al.: "Membrane-bound D-glucose dehydrogenase from Pseudomonas sp.; Solubilization, purification and characterization" * Whole document *<br>--- | 1,3-5, 7-11, 13,14, 18,19, 21-26 | C 12 Q 1/54<br>C 12 N 9/04 |
| Y | CHEMICAL ABSTRACTS, vol. 92, no. 13, 31st March 1980, page 263, no. 106368r, Columbus, Ohio, USA J.A. DUINE et al.: "Glucose dehydrogenase from Acinetobacter calcoaceticus. A "quinoprotein"" & FEBS LETT. 1979, 108(2), 443-446 * Abstract *<br>--- | 1,5,6, 19,20 | |
| | | | TECHNICAL FIELDS SEARCHED (Int. Cl. 3) |
| Y | CHEMICAL ABSTRACTS, vol. 96, no. 23, 7th June 1982, page 371, no. 196306d, Columbus, Ohio, USA J.A. DUINE et al.: "Different forms of quinoprotein aldose-(glucose-)dehydrogenase in Acinetobacter calcoaceticus" & ARCH. MICROBIOL. 1982, 131(1), 27-31 * Abstract *<br>--- -/- | 1,5,6, 19,20 | C 12 Q<br>C 12 N |

The present search report has been drawn up for all claims

| Place of search | Date of completion of the search | Examiner |
|---|---|---|
| THE HAGUE | 16-08-1983 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

0094161

European Patent Office

**EUROPEAN SEARCH REPORT**

Application number

EP 83 30 2145



**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| Y | CHEMICAL ABSTRACTS, vol. 95, no. 17, 26th October 1981, page 239, no. 145988z, Columbus, Ohio, USA M. AMEYAMA et al.: "Existence of a novel prosthetic group, PQQ, in membrane-bound, electron transport chain-linked, primary dehydrogenases of oxidative bacteria" & FEBS LETT. 1981, 130(2), 179-183 * Abstract * --- | 1,5,19 | |
| Y | CHEMICAL ABSTRACTS, vol. 90, no. 21, 21st May 1979, page 86, no. 162572y, Columbus, Ohio, USA B. WURTZ: "The effecxts of dichlorophenol-indophenol on the glucose dehydrogenase activity of Pseudomonas fluorescens (type R.)" & C.R. SEANCES SOC. BIOL. SES. FIL. 1978, 172(4), 744-747 * Abstract * --- | 1,5,9, 10,11, 18,19, 23-25 | TECHNICAL FIELDS SEARCHED (Int. Cl. 3) |
| Y | US-A-4 120 755 (K.J. PIERRE et al.) * Whole document * --- | 1,3,18 | |
| Y | US-A-3 964 974 (D. BANAUCH et al.) * Columns 7,8; examples 1-7 * --- -/- | 1-4,9, 10,12, 13,18, 23,24 | |

The present search report has been drawn up for all claims

| Place of search | Date of completion of the search | Examiner |
|---|---|---|
| THE HAGUE | 16-08-1983 | GRIFFITH G. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0094161

European Patent Office

# EUROPEAN SEARCH REPORT

Application number

EP 83 30 2145



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-3 506 544 (H.P. SILVERMAN et al.)<br>* Whole document *<br>--- | 1,14-16,18,26 | |
| A | US-A-2 999 052 (H.G. ALBAUM et al.)<br>--- | | |
| A | EP-A-0 029 104 (MILES LABORATORIES INC.)<br>--- | | |
| A | US-A-3 009 862 (L.A. DOBRICK)<br>--- | | |
| A | GB-A-1 340 848 (F. HOFFMANN-LA ROCHE & CO.)<br>----- | | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 16-08-1983 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82